# EUROPEAN PATENT APPLICATION

(11) **EP 4 740 833 A1**
(43) Date of publication of application: **13.05.2026**
(21) Application number: 25212922.6
(22) Date of filing: 03.11.2025
(51) Int. Cl.: A61B 3/00, A61B 3/032

(54) **OPHTHALMOLOGIC APPARATUS AND METHOD FOR CONTROLLING THE SAME**

(30) Priority: 11.11.2024 JP 2024196949
(71) Applicant: Topcon Corporation, Tokyo 174-8580 (JP)
(72) Inventor: SAKURADA, Tomohiro, Tokyo, 174-8580 (JP)
(74) Representative: Wagner, Jürgen

(57) **Abstract**

An ophthalmologic apparatus (10) includes a visual target presentation portion (21, 12) that is configured to present a visual target (25) to a subject eye and to adjust a presentation mode of the visual target (25); an increase and decrease operation portion (33) that is configured to make an increase operation to increase a numerical value and a decrease operation to decrease the numerical value; a control portion (14) that is configured to change the presentation mode of the visual target (25) presented by the visual target presentation portion (21, 12) in response to the increase operation or the decrease operation on the increase and decrease operation portion (33); and a direction notification portion (35) that is configured to notify an increase direction for the increase operation or a decrease direction for the decrease operation on the increase and decrease operation portion (33). The control portion (14) is further configured to set a predicted value (Vp) for a presentation mode that is predicted to be appropriate for the subject eye; to set an operation starting value (Vs) for a presentation mode that is different from the predicted value (Vp); to set the increase and decrease operation portion (33) to the operation starting value (Vs); and to control the direction notification portion (35) to notify an operation direction (Do) from the operation starting value (Vs) toward the predicted value (Vp).

## Description

### FIELD OF THE INVENTION

The present invention relates to an ophthalmologic apparatus.

### BACKGROUND

It is known in the art that an ophthalmologic apparatus enables examination of visual functions of subject eyes by allowing an examinee to adjust the visibility of a visual target presented to the subject eyes through his or her own operation (see, for example, JP2009-082581A). This ophthalmologic apparatus enables the examinee to obtain satisfactory examination results by checking the visibility of the visual target, which changes according to the operation on an operation portion of the apparatus, and adjusting the visibility so that the visual target can be seen most appropriately.

### SUMMARY

The conventional ophthalmologic apparatus may lead to differences in both the time required to select the most appropriate visibility of the visual target by operating the operation portion and the accuracy of the selection depending on factors such as the examinee's experience and familiarity with the examination.

The present invention has been made in view of the above circumstances. An object of the present invention is to provide an ophthalmologic apparatus that enables the examinee to obtain examination results quickly and appropriately by operating the operation portion.

The present invention provides an ophthalmologic apparatus including: a visual target presentation portion that is configured to present a visual target to a subject eye and to adjust a presentation mode of the visual target; an increase and decrease operation portion that is configured to perform an increase operation to increase a numerical value and a decrease operation to decrease the numerical value; a control portion that is configured to change the presentation mode of the visual target presented by the visual target presentation portion in response to the increase operation or the decrease operation on the increase and decrease operation portion; and a direction notification portion that is configured to notify an increase direction for the increase operation or a decrease direction for the decrease operation on the increase and decrease operation portion. The control portion is further configured to set a predicted value for a presentation mode that is predicted to be appropriate for the subject eye; to set an operation starting value for a presentation mode that is different from the predicted value; to set the increase and decrease operation portion to the operation starting value; and to control the direction notification portion to notify an operation direction from the operation starting value toward the predicted value.

The ophthalmologic apparatus with the above configuration can quickly obtain appropriate examination results through the examinee's operation on the operation portion.

### BRIEF DESCRIPTION OF DRAWINGS

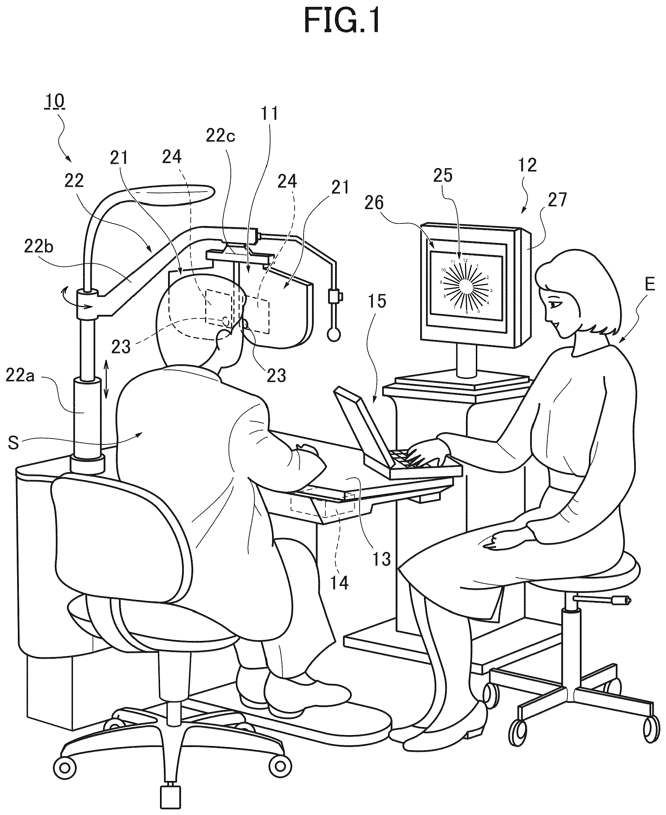

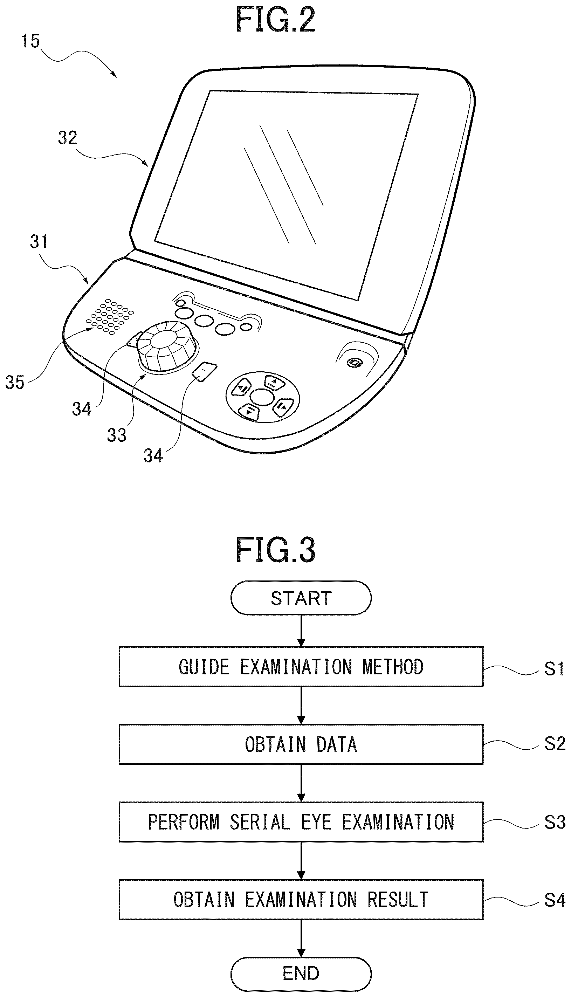

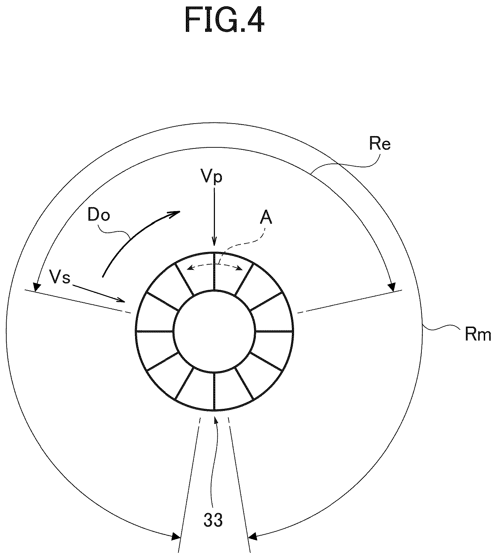

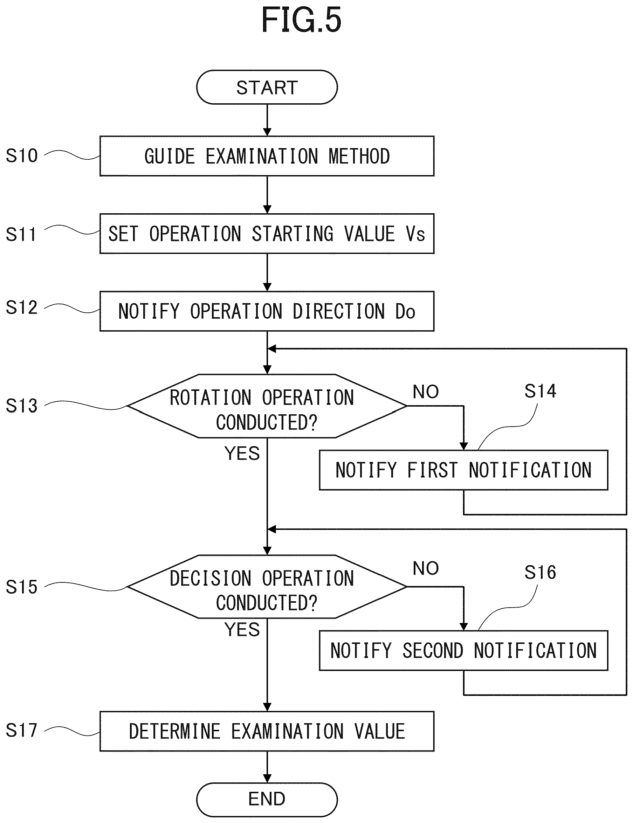

FIG. 1 is a schematic view of an ophthalmologic apparatus according to a first embodiment. FIG. 2 is a schematic view of a controller of the ophthalmologic apparatus. FIG. 3 is a flowchart of a sequential eye examination process using a controller of the first embodiment. FIG. 4 is a schematic view showing a predicted value, an operation starting value, a maximum adjustment range, an operation direction, and an effective adjustment range in a rotation operation of a rotation operation portion. FIG. 5 is a flowchart of a self-examination process using the controller of the first embodiment.

### DETAILED DESCRIPTION

With respect to the use of plural and/or singular terms herein, those having skill in the art can translate from the plural to the singular and/or from the singular to the plural as is appropriate to the context and/or application. The various singular/plural permutations may be expressly set forth herein for the sake of clarity.

An embodiment of the ophthalmologic apparatus according to the present invention is described with reference to FIGS. 1 to 5. FIG. 4 schematically shows a rotation operation portion 33 together with a predicted value Vp, an operation starting value Vs, a maximum adjustment range Rm, an operation direction Do, and an effective adjustment range Re. These are shown in a simplified manner to facilitate understanding and may not exactly correspond to the actual state of the rotation operation portion 33.

An ophthalmologic apparatus 10 according to the first embodiment is an example of a subjective ophthalmologic apparatus used for subjective examinations of subject eyes. This ophthalmologic apparatus 10 presents various visual targets 25 to an examinee S and enables subjective examination of visual functions based on responses from the examinee S regarding his or her perception of the visual targets. The ophthalmologic apparatus 10 of the first embodiment is a binocular open-type apparatus that enables examination and correction of the visual functions of subject eyes while the examinee S keeps both eyes open. The ophthalmologic apparatus 10 also enables examination of one of the eyes while the other eye is shielded.

As shown in FIG. 1, the ophthalmologic apparatus 10 includes a refractor head (measuring head) 11, a visual target display device 12, an optometric table 13, a control portion 14, and a controller 15. The refractor head 11, the visual target display device 12, the control portion 14, and a controller 15 are connected through a general communication interface (I/F) to allow communication therebetween. The optometric table 13 supports the refractor head 11 and allows the examinee's arms and the controller 15 to be placed thereon. The optometric table 13 is movable in the vertical direction and can be fixed at any desired vertical position.

The refractor head 11 includes a pair of eye examination portions 21 and a support mechanism 22. The pair of eye examination portions 21 is attached to the optometric table 13 through the support mechanism 22. The support mechanism 22 includes a pillar 22a, a support arm 22b, and a support member 22c. The pillar 22a extends vertically upward from the optometric table 13. The pillar 22a is extendable in the vertical direction and rotatable in the circumferential direction, as indicated with the arrows in FIG. 1. The support arm 22b extends diagonally upward from the pillar 22a. The support member 22c is provided at the distal end of the support arm 22b. The refractor head 11 is suspended from the support member 22c. The rotation of the pillar 22a in the circumferential direction enables the refractor head 11 to be positioned between the examinee S and the visual target display device 12 or retracted from that position.

The eye examination portions 21 are provided as a pair on the left and right sides to correspond to the left and right subject eyes of the examinee S and individually constitute an eye examination optical system for the left eye and an eye examination optical system for the right eye. The eye examination portions 21 are slidably attached to the support member 22c of the support mechanism 22 to be moved toward and away from each other. This enables each eye examination portion 21 to be positioned relative to the left and right subject eyes of the examinee S. Each of the eye examination portions 21 includes an eye examination window 23. The eye examination window 23 forms an optical path from the subject eye side of the corresponding eye examination portion 21 toward the visual target display device 12.

Each of the eye examination portions 21 houses an optical element portion 24. The optical element portion 24 enables a suitable optical member for eye examinations to be selectively disposed in the optical path through the eye examination window 23 of the eye examination portion 21. The optical members include polarizing members and various lenses for correcting the visual functions of the subject eyes, for example, a polarizing filter, a spherical lens, a cylindrical lens, and a prism. Each of the optical element portions 24 includes a plurality of turret discs, each provided with optical members. Each of the turret discs is rotatable and stoppable at any position so that a selected optical member can be positioned to face the eye examination window 23. Each of the eye examination portions 21 presents the visual targets 25 from the visual target display device 12 to the left and right subject eyes of the examinee S through the eye examination window 23 with the adjusted optical element portion 24. The presentation is made under selected optical characteristics.

The eye examination portion 21 is not limited to the configuration of the first embodiment as long as it can arrange the optical members in front of the subject eye and between the subject eye and the visual target display device 12. For example, the eye examination portion 21 may use a liquid lens whose optical characteristics can be changed by applying voltage or other means.

The visual target display device 12 is disposed in front of the subject eyes with the refractor head 11 positioned therebetween. The visual target display device 12 is configured to display the visual target 25 presented to the examinee S. The visual target display device 12 includes a display device body 27 provided with a display window 26 in which the visual target 25 is displayed. The display device body 27 houses a visual target optical system that generates a visual target image for examining the visual functions of the examinee S when the examinee S views an object. The visual target optical system causes the visual targets 25 to be displayed at desired examination distances through the display window 26. For example, the visual target 25 displayed on an electronic display device is formed into a virtual image at an image point position corresponding to any desired examination distance through optical elements such as lenses and mirrors. The electronic display device may be, for example, a liquid crystal display (LCD) or an organic EL (OLED) display.

The visual target optical system varies the examination distance, so that it can display the visual target 25 at various examination distances. The visual target optical system can also change the design or symbol displayed on the electronic display device, thereby changing the visual target 25. The visual targets 25 are used for subjective examinations and include, for example, visual acuity targets, red-green examination targets, astigmatism examination targets, binocular visual function examination targets, Landolt rings, Snellen charts, and E charts. The visual targets 25 may also include, for example, letters such as hiragana, katakana, or alphabet letters; pictures of animals or fingers; specific figures for binocular visual function examinations such as cross-shape targets; and landscape paintings or landscape photographs. These visual targets 25 may be still images or moving pictures.

The visual target display device 12, under the control of the control portion 14, can change the design or symbol used as the visual target 25 and the examination distance for displaying the visual target 25. Thus, the visual target display device 12 can display the visual target 25 with any design or symbol at any examination distance through the display window 26 of the display device body 27. The examination distance is not the actual distance between the subject eye and the visual target 25, but an apparent distance created with the optical element portion 24 of the eye examination portion 21, as if the visual target 25 were presented at that distance.

Thus, the ophthalmologic apparatus 10 causes the visual target display device 12 to display the visual target 25 for a desired eye examination and adjusts the optical element portions 24 of the left and right eye examination portions 21 of the refractor head 11, thereby presenting the visual target 25 to the subject eyes at any examination distance. The ophthalmologic apparatus 10 also adjusts the optical characteristics of the optical element portions 24 of the eye examination portions 21 and adjusts the type and orientation of the visual target 25 displayed on the visual target display device 12, thereby changing the presentation mode of the visual target 25. Therefore, in the ophthalmologic apparatus 10, the two eye examination portions 21 of the refractor head 11 and the visual target display device 12 function together as a visual target presentation portion that presents the visual target 25 to the subject eyes and adjusts the presentation mode of the visual target 25.

The control portion 14 controls the overall operation of each part of the ophthalmologic apparatus 10. According to a predetermined program or an operation on the separate controller 15, the control portion 14 sets the optical characteristics in the refractor head 11, sets the design or symbol of the visual target 25 displayed on the visual target display device 12, and sets the examination distance of the visual target 25. The control portion 14 controls the liquid crystal display portion 32 of the separate controller 15, which will be described later, to display a menu screen, an examination result screen and/or the visual target 25 to be presented. Furthermore, the control portion 14 may control the rotation operation portion 33 to display a predicted value Vp, an operation starting value Vs, a maximum adjustment range Rm, an operation direction Do, and/or an effective adjustment range Re, which will be described later. The control portion 14 also controls a voice output portion 35 of the separate controller 15, which will be described later, to output voice. Although the control portion 14 is provided in the optometric table 13 in FIG. 1, its location is not limited to the one in the first embodiment as long as it controls the elements described in this specification.

As shown in FIG. 2, an examiner E or an examinee S uses the controller 15 to operate the ophthalmologic apparatus 10. The controller 15 is configured to exchange signals and data with the control portion 14. The controller 15 includes a controller body 31 and a liquid crystal display portion 32 connected thereto. The controller body 31 includes a plurality of switches and other elements to enable operation of the refractor head 11 and the visual target display device 12. The controller body 31 of the first embodiment includes at least a rotation operation portion 33, a determination operation portion 34, and a voice output portion 35.

The rotation operation portion 33 is configured to input a numerical value with a rotation operation. The rotation operation portion 33 is used, for example, to change the numerical value of the refractive power of a lens or the astigmatic axis and can be turned clockwise to decrease the value or counterclockwise to increase the value. The rotation operation portion 33 can finely adjust a numerical value in step increments through the rotation operation which gradually changes the numerical value, each step corresponding to a predetermined amount of change. Thus, the rotation operation portion 33 functions as an increase and decrease operation portion that increases or decreases a numerical value. The rotational position of the rotation operation portion 33 corresponds to the operation position of the increase and decrease operation portion. The rotation operation portion 33 transmits a signal to the control portion 14 in accordance with the degree (number of steps) of the rotation operation. The control portion 14 changes the presentation mode of the visual target 25 in accordance with the degree (number of steps) of the rotation operation of the rotation operation portion 33. For example, in adjusting the refractive power of a lens, the control portion 14 changes the optical elements of the eye examination portions 21 in accordance with the degree (number of steps) of the rotation operation, thereby adjusting the refractive power of the lens. For example, in adjusting the astigmatic axis, the control portion 14 changes the direction or type of the visual target 25 displayed on the electronic display device of the visual target display device 12 in accordance with the degree (number of steps) of the rotation operation, thereby adjusting the astigmatic axis. The rotation operation portion 33 is not limited to the configuration in the first embodiment, and as long as it employs a rotation operation, the operational manner, position, and configuration of the rotation operation portion 33 may be set as appropriate.

The determination operation portion 34 is pressed to determine a value adjusted through the rotation operation of the rotation operation portion 33 (hereinafter referred to as a "determination operation"). The determination operation portion 34 transmits a signal corresponding to the determination operation to the control portion 14. For example, when a determination operation is made on the determination operation portion 34, the control portion 14 determines that the refractive power of the lens, the astigmatic axis, or other examination items indicated by using the rotation operation portion 33 at that time have been selected. Thus, when operated together with the rotation operation portion 33, the determination operation portion 34 facilitates adjustment of the refractive power of the lens, the astigmatic axis, and the other examination items. In the first embodiment, the determination operation portions 34 are provided as a pair with the rotation operation portion 33 located therebetween. Thus, even when the examiner E or the examinee S rotates the rotation operation portion 33 with one of the left or right hands, the determination operation can be made with the other hand. The determination operation portion 34 is not limited to the configuration of the first embodiment, and as long as it allows a determination operation for confirming a value adjusted through the rotation operation, the operational manner, position, and structure of the determination operation portion 34 may be set as appropriate.

The controller 15 is configured to allow various operations in addition to those using the rotation operation portion 33 and the determination operation portion 34. The switches of the controller 15 can be used for selecting an eye examination program for various measurements of the subject eyes, selecting either one eye or both eyes as the examination target, starting the selected eye examination program, ending the measurement, and for other operations. These operations are made, for example, through switches provided alongside the rotation operation portion 33 and/or the determination operation portion 34 as shown in FIG. 2, through operation icons displayed on the liquid crystal display portion 32, or through voice input via a voice recognition portion.

The voice output portion 35 is configured to output guidance voice regarding the operation of examinations using the ophthalmologic apparatus 10 and the other operations. The voice output portion 35 outputs predetermined voice under the control of the control portion 14. In the first embodiment, the voice output portion 35 outputs various voice outputs according to operations on the rotation operation portion 33 and the determination operation portion 34 (see FIG. 5). The voice output portion outputs at least a guidance notification (see Step S10 in FIG. 5), a first notification (see Step S14 in FIG. 5), and a second notification (see Step S16 in FIG. 5), which will be described later.

The liquid crystal display portion 32 is configured with an electronic display device such as an electroluminescence (EL) display or a liquid crystal display (LCD) and displays any desired image under the control of the control portion 14. The liquid crystal display portion 32 appropriately displays a menu screen, examination parameters, examination information, examination results, the visual target 25, and other information. The liquid crystal display portion 32 may also display operation icons for various operations. In this case, the liquid crystal display portion 32 may include a touch panel function to enhance operability. Furthermore, the liquid crystal display portion 32 can display designs or texts for operation guidance during execution of the selected eye examination program.

As long as the controller 15 includes the rotation operation portion 33 that allows the rotation operation and the determination operation portion 34 that allows the determination operation, it does not need to include other operation portions. The controller 15 may be configured with a notebook computer (personal computer) or a portable terminal (information processing device) such as a tablet terminal or a smartphone. As long as the voice output portion 35 can notify the examinee S of a guidance notification, a first notification, a second notification, and other notifications, it may be provided in the refractor head 11, the visual target display device 12, or the optometric table 13, or may be provided separately from them. The configuration of the voice output portion 35 is not limited to that in the first embodiment.

The ophthalmologic apparatus 10 examines eyes as follows. First, as shown in FIG. 1, the support mechanism 22 of the ophthalmologic apparatus 10 is rotated as desired, and the refractor head 11 is positioned in front of the display window 26 of the visual target display device 12. While the examinee S is seated in a chair, he or she takes an eye examination posture facing the display window 26 of the visual target display device 12 with the refractor head 11 located therebetween. The height of the refractor head 11 and the visual target display device 12 is adjusted to match the height of the subject eyes of the examinee S. The pillar 22a is extended or contracted to finely adjust the height of the eye examination portions 21 relative to the subject eyes.

Then, the visual target display device 12 displays a visual target 25 suitable for the purpose of the examination. The ophthalmologic apparatus 10 adjusts each of the optical element portions 24 of the left and right eye examination portions 21, so that the visual target display device 12 presents the visual target 25 at a desired examination distance. The ophthalmologic apparatus 10 examines the subject eyes at the desired examination distance by having the examinee S gaze at the visual target image through the eye examination windows 23 of the left and right eye examination portions 21. The ophthalmologic apparatus 10 can obtain examination results for correcting refractive errors, heterophoria, and other visual abnormalities under a condition where the examinee S gazes at the visual target image at the desired distance, by adjusting the refractive power of lenses and/or the power of prisms of the optical element portions 24 in the refractor head 11 in accordance with the way in which the examinee S perceives the visual target 25.

Next, a sequential eye examination process in the ophthalmologic apparatus 10 is described with reference to the flowchart shown in FIG. 3. The control portion 14 executes the sequential eye examination process based on a program stored in a storage such as an internal memory. The process shown in the flowchart of FIG. 3 starts when a sequential eye examination mode is selected in the ophthalmologic apparatus 10.

In Step S1, guidance on the examination method is provided, and then the process proceeds to Step S2. In Step S1, the liquid crystal display portion 32 of the controller 15 displays, in the first embodiment, a video that includes information such as the type of examination to be conducted, an outline of each examination, and precautions for each examination. The examinee S can understand the procedure of various examination methods by viewing the video. Alternatively, the guidance on the examination method may be provided by displaying the video on another display or by outputting the voice guidance through the voice output portion 35.

In Step S2, data on the examinee S is obtained and then, the process proceeds to Step S3. In Step S2, various data on the subject eyes of the examinee S is obtained. The data may include, for example, the results of the previous examination.

In Step S3, the sequential eye examination is executed, and then the process proceeds to Step S4. In Step S3, various types of examinations of the subject eyes are executed in sequence. For example, the examinations include a spherical power examination which includes a spherical test, a distance vision test, and a red-green test; astigmatic axis angle and astigmatism power examinations which use a cross cylinder; a binocular balance examination to make the accommodation of the left and right eyes substantially equal; and an add power examination to measure the power difference between distance power and near power. The order of these examinations may be as described above or may be different. In Step S3, the examinations may be appropriately selected from among these examples, and other examinations may also be included as needed. The sequential eye examination is not limited to the above examples.

In Step S4, the control portion 14 obtains the examination results and ends the sequential eye examination process. In Step S4, the control portion 14 obtains the examination results from various examinations conducted in Step S3. In Step S4, the control portion 14 displays the obtained examination results on the liquid crystal display portion 32 of the separate controller 15.

Consequently, the ophthalmologic apparatus 10 can conduct various examinations on the subject eyes. The various examinations described above are subjective eye examinations based on the examinee's perception. Therefore, it is desirable for the ophthalmologic apparatus 10 to allow the examinee S to make an adjustment so that he or she can see best. Thus, the ophthalmologic apparatus 10 of the present invention is configured to allow the examinee S to make an adjustment of visual perception by using the rotation operation portion 33 and the determination operation portion 34 of the separate controller 15. This is explained with reference to FIG. 4.

First, the control portion 14 in the ophthalmologic apparatus 10 sets the data obtained in Step S2 of the sequential eye examination process as a predicted value Vp and also sets a value deviating from the predicted value Vp as an operation starting value Vs. The predicted value Vp is based on a previous examination. Thus, the predicted value Vp is considered to be close to the actual examination value of the subject eye. The value deviating from the predicted value Vp refers to a value that provides visibility clearly different from that of the predicted value Vp and that is closer to the predicted value Vp than the limit value of the maximum adjustment range Rm that can be adjusted using the rotation operation portion 33. The examinee S can reliably perceive the change in visibility while the time required to approach the predicted value Vp is shortened when the value is adjusted from the operation starting value Vs toward the predicted value Vp. As shown in FIG. 4, the operation starting value Vs is set on the left side of the predicted value Vp in the rotation direction of the rotation operation portion 33, but it may alternatively be set on the right side. The setting is not limited to the example shown in FIG. 4.

After setting the operation starting value Vs, the control portion 14 controls the voice output portion 35 to notify the operation direction Do from the operation starting value Vs toward the predicted value Vp. In the example shown in FIG. 4, the control portion 14 controls the voice output portion 35 to notify the operation direction Do as a voice message such as "Please rotate the rotation operation portion 33 clockwise." Thus, in the first embodiment, the voice output portion 35 functions as a direction notification portion that notifies the examinee S of the operation direction Do from the operation starting value Vs toward the predicted value Vp. The operation direction Do indicates either an increase direction or a decrease direction, which specifies whether the operation on the increase and decrease operation portion causes the value to increase or to decrease. The direction notification portion is not limited to the configuration of the first embodiment, as long as it notifies the examinee S of the operation direction Do (increase direction or decrease direction). Since the examinee S views the visual target through the eye examination window 23 of each of the eye examination portions 21 during the examination, it is desirable that the notification can be provided while the examinee S maintains this state. For example, the operation direction Do may be displayed as a symbol such as an arrow or a letter around the visual target 25 displayed on the visual target display device 12.

Furthermore, the control portion 14 sets an effective adjustment range Re based on the predicted value Vp. The effective adjustment range Re is adjustable through the rotation operation of the rotation operation portion 33. In other words, when the effective adjustment range Re is set, the rotation operation portion 33 prevents the value from exceeding this range. The effective adjustment range Re may restrict the physical rotation of the rotation operation portion 33 or may limit only the change in value without restricting the physical rotation. The effective adjustment range Re includes at least the predicted value Vp and is smaller than the maximum adjustment range Rm. In the example shown in FIG. 4, the effective adjustment range Re includes the operation starting value Vs. However, the effective adjustment range Re may not include the operation starting value Vs. In the latter case, when the rotation operation portion 33 is rotated from the operation starting value Vs toward the predicted value Vp, control may start to limit the adjustable range of the rotation operation to the effective adjustment range Re after the value indicated by using the rotation operation portion 33 enters the effective adjustment range Re.

After the guidance for starting the corresponding examination is provided, if no rotation operation is made on the rotation operation portion 33 even after a predetermined time elapses, the control portion 14 controls the voice output portion 35 to output a first notification to prompt the rotation operation. The first notification may include a message, for example, "Please rotate the rotation operation portion 33 until the visual target 25 becomes clearly visible" or "Please rotate the rotation operation portion 33 to find a position where the visual target 25 is clearly visible." The first notification is not limited to the above examples and may be appropriately set according to the content of examination as long as the first notification prompts the rotation operation.

If the determination operation portion 34 does not receive any determination operation even after a lapse of a predetermined time following the rotation operation on the rotation operation portion 33, the control portion 14 controls the voice output portion 35 notify the second notification to prompt the determination operation. For example, in a situation where an adjustment is required so that the visual target 25 appears the same at two examination values arranged in sequence by switching the rotation operation, the appearances at the two examination values may not actually be the same but may instead be reversed from each other. In such a situation, since the appearances at the two examination values are not the same, the examinee S may become confused while repeating the rotation operation on the rotation operation portion 33. Assuming such a situation, the second notification may include, for example, a message that eliminates confusion of the examinee S, such as "Please perform the determination operation when the appearances at the two examination values arranged in sequence by switching the rotation operation have become approximately the same." The second notification is not limited to the above examples and may be appropriately set according to the content of examination as long as the second notification prompts the determination operation.

Thus, the voice output portion 35 also functions as an operation notification portion that notifies the examinee S of the operation for examination in the first embodiment. The operation notification portion is not limited to the configuration of the first embodiment as long as it notifies the examinee S of the first and second notifications. Since the examinee S views the visual target through the eye examination window 23 of each of the eye examination portions 21 during the examination, it is desirable that the notification can be provided while the examinee S maintains this state. For example, the first and second notifications may be presented as character information around the visual target 25 displayed on the visual target display device 12.

Next, a self-examination process in the ophthalmologic apparatus 10 will be described with reference to the flowchart shown in FIG. 5. In the self-examination, the examinee S conducts a visual function examination of the subject eyes alone by using the ophthalmologic apparatus 10. The self-examination process may be executed when one examination is selected, or sequentially in correspondence with the examinations conducted in Step S3 of the sequential eye examination process shown in FIG. 3. The control portion 14 executes the self-examination process based on a program stored in a storage such as an internal memory.

In Step S10, guidance on the examination method is provided, and the process proceeds to Step S11. In Step S10 of the first embodiment, a video is displayed on the liquid crystal display portion 32 of the controller 15. The video shows an outline of the examination, precautions, and a method of operating the rotation operation portion 33 with respect to how the visual target 25 appears. By viewing the video, the examinee S can understand the operations during the examination and how the visual target 25 is perceived. The guidance on the examination method may alternatively be provided by displaying the video on another display or outputting the voice guidance through the voice output portion 35.

In Step S11, an operation starting value Vs is set, and the process proceeds to Step S12. In Step S11, previous data of the same examination on the subject eyes of the examinee S is obtained and set as a predicted value Vp, and a value deviating from it is set as the operation starting value Vs. The effective adjustment range Re including the predicted value Vp is also set in Step 11. Accordingly, Step S11 serves as a step for setting the predicted value Vp and the operation starting value Vs.

In Step S12, an operation direction Do is notified, and the process proceeds to Step S13. In Step S12, the control portion 14 controls the voice output portion 35, which functions as the direction notification portion, to output the operation direction Do from the operation starting value Vs toward the predicted value Vp. After Step S12, the presentation mode of the visual target 25 changes according to the degree (number of steps) of the rotation of the rotation operation portion 33.

In Step S13, it is determined whether the rotation operation has been made. If YES (i.e., the rotation operation has been made), the process proceeds to Step S15. If NO (i.e., the rotation operation has not been made), the process proceeds to Step S14. In other words, it is determined whether the rotation operation portion 33 has been operated in Step S13.

In Step S14, a first notification is output, and the process returns to Step S13. In Step S14, when no rotation operation is made on the rotation operation portion 33 even after a predetermined time elapses following the notification of the operation direction Do in Step S12, the control portion 14 controls the voice output portion 35 to output a first notification to prompt the rotation operation.

In Step S15, it is determined whether the determination operation has been made. If YES (i.e., the determination operation has been made), the process proceeds to Step S17. If NO (i.e., the determination operation has not been made), the process proceeds to Step S16. In Step S15, it is determined whether the determination operation portion 34 has been operate in Step S13.

In Step S16, a second notification is output, and the process returns to Step S15. In Step S16, when no determination operation is made on the determination operation portion 34 even after a predetermined time elapses following the determination in Step S13 that the rotation operation has been made, the control portion 14 controls the voice output portion 35 to output a second notification to prompt the determination operation.

In Step S17, an examination value is determined, and the self-examination process is completed. In Step S17, when the determination operation is performed on the determination operation portion 34, the control portion 14 determines, as the examination value, the value indicated by using the rotation operation portion 33 at the time of the determination operation.

In the above self-examination process, when Step S14 and/or Step S16 are repeated multiple times, the self-examination process may be terminated if a preset upper limit of the elapsed time or the number of repetitions for these steps is reached. This is because, if the first and/or second notification does not resolve the issue, it is appropriate to temporarily terminate the self-examination process.

Next, each step of the flow of the self-examination process is described. Once the examinee S faces the refractor head 11 and the face is positioned at a predetermined location, and a predetermined examination of the subject eyes starts. The process proceeds in the order of Steps S10, S11, and S12. The control portion 14 provides guidance on the examination method, sets an operation starting value Vs and an effective adjustment range Re. Then, the control portion 14 controls the voice output portion 35 to output an operation direction Do.

While viewing the visual target 25 presented via the refractor head 11 and the visual target display device 12, the examinee S rotates the rotation operation portion 33 in the indicated operation direction Do to adjust the appearance of the visual target 25 as instructed. For example, in a spherical power examination or an astigmatism power examination, the control portion 14 enables adjustment of the appearance of the visual target 25 by changing optical elements of the eye examination portions 21 of the refractor head 11 in response to the rotation operation. This corresponds to an exemplary step for adjusting the presentation mode of the visual target by adjusting the optical setting for the subject eye in the eye examination portion. In an astigmatic axis angle examination, the control portion 14 enables adjustment of the appearance of the visual target 25 by changing the orientation, in the rotational direction, of the visual target 25 displayed on the visual target display device 12 and/or optical elements of the eye examination portions 21 in response to the rotation operation. This corresponds to an exemplary step for adjusting the presentation mode by adjusting the orientation of the visual target displayed on the visual target display device.

When the appearance of the visual target 25 matches the instructed condition, the examinee S stops rotating the rotation operation portion 33 and performs a determination operation on the determination operation portion 34. The process then proceeds through Steps S13, S15, and S17. The control portion 14 determines, as the examination value, the value indicated by using the rotation operation portion 33 when the determination operation is made. In this manner, a value at which the visual target 25 is appropriately seen by the examinee S is adopted as the examination value, allowing the examinee S to obtain a result that is satisfactory.

If no rotation operation is made on the rotation operation portion 33 even after a predetermined time elapses following Step S12, the process proceeds to Step S14. The control portion 14 controls the voice output portion 35 to output a first notification that prompts the rotation operation. Thus, the ophthalmologic apparatus 10 informs the examinee S that this is the stage for adjustment by rotating the rotation operation portion 33, thereby facilitating the examination even when the examinee S is uncertain about what action to take or when to start the adjustment.

If no determination operation is made on the determination operation portion 34 even after a predetermined time elapses following Step S14, the process proceeds to Step S16. The control portion 14 controls the voice output portion 35 to output a second notification that prompts the determination operation. Thus, the ophthalmologic apparatus 10 informs the examinee S that this is the stage to determine the examination value through the determination operation on the determination operation portion 34 and helps the examinee S recognize an appropriate endpoint of the rotation-based adjustment. As a result, the ophthalmologic apparatus 10 facilitates the examination even when the examinee S does not know how to determine the value or has forgotten to make the determination operation.

In conventional ophthalmologic apparatuses, the starting point for adjustment might be set near a previous examination result to ease the examinee's rotation-based adjustment. However, such a configuration can make it difficult for the examinee to recognize changes in the appearance of the visual target, leading to back-and-forth adjustment around the starting value and making it harder to select a position that provides an appropriate appearance (see arrow A in FIG. 4). This tendency is pronounced for examinees with little knowledge of, or little familiarity with, the examination.

In contrast, in the ophthalmologic apparatus 10, the control portion 14 sets a value deviating from the predicted value Vp as the operation starting value Vs, uses this value as the starting point of the rotation operation on the rotation operation portion 33, and controls the voice output portion 35 to output the operation direction Do from the operation starting value Vs toward the predicted value Vp. Consequently, the examinee S reliably recognizes changes in the appearance of the visual target 25 through the rotation operation and perceives the degree of change in relation to the degree of rotation. The examinee S can thus reliably identify a value at which the visual target 25 is appropriately seen and can readily adjust to that value through the rotation operation. Therefore, even an examinee S with little knowledge or experience in the examination can reliably and quickly determine an appropriate examination value by operating the rotation operation portion 33.

Moreover, the control portion 14 sets an effective adjustment range Re that includes the predicted value Vp. Thus, the ophthalmologic apparatus 10 prevents adjustment from straying far from a value at which the visual target is appropriately seen due to the examinee's rotation operation, thereby avoiding difficulty in returning to an appropriate value. As a result, the ophthalmologic apparatus 10 can determine an appropriate examination value more reliably and quickly through the examinee's rotation operation of the rotation operation portion 33.

Furthermore, as long as the ophthalmologic apparatus 10 changes the presentation mode of the visual target 25 in response to the rotation operation of the rotation operation portion 33 under the control of the control portion 14, the present invention can be applied simply by setting the operation starting value Vs for the rotation operation portion 33. Accordingly, the apparatus can be implemented with minimal additional elements, enabling effective use of existing ophthalmologic apparatuses.

The examinee S can intuitively adjust the examination value by rotating the rotation operation portion 33. In addition, since the examination value is determined by using the determination operation, which is independent of the rotation operation, on the determination operation portion 34, the resulting examination value appropriately reflects the examinee's intention. In other words, the ophthalmologic apparatus 10 requires two separate and functionally different stages of operation: the adjustment operation, in which the rotation operation portion 33 is rotated, and the determination operation, in which the determination operation portion 34 is pressed to determine the adjustment. Therefore, the examinee S can intuitively adjust and appropriately determine the examination value with simple operations.

The ophthalmologic apparatus 10 of the first embodiment according to the present invention can bring the following advantageous effects. The ophthalmologic apparatus 10 includes the eye examination portion 21 and the visual target display device 12, as a visual target presentation portion that is configured to present a visual target 25 to a subject eye and to adjust a presentation mode of the visual target 25, and the rotation operation portion 33 (increase and decrease operation portion) that is configured to make a rotation operation as an increase operation to increase a numerical value and a decrease operation to decrease the numerical value. The ophthalmologic apparatus 10 further includes a control portion 14 that is configured to change the presentation mode of the visual target 25 presented by using the eye examination portion 21 and the visual target display device 12 in response to the rotation operation on the rotation operation portion 33, and the voice output portion 35 as the direction notification portion that is configured to notify a direction of the rotation operation on the rotation operation portion 33. The control portion 14 is configured to set a predicted value Vp for a presentation mode of the visual target 25 that is predicted to be appropriate for the subject eye, and an operation starting value Vs for a presentation mode of the visual target 25 that is different from the predicted value Vp. The control portion 14 sets the rotation operation portion 33 to the operation starting value Vs and controls the voice output portion 35 to notify the operation direction Do from the operation starting value Vs toward the predicted value Vp. Therefore, the ophthalmologic apparatus 10 allows an examinee S to surely recognize a value at which the visual target 25 is appropriately seen and facilitates adjustment to obtain the value at which it is appropriately seen through the examinee's rotation operation.

In the ophthalmologic apparatus 10, the control portion 14 is configured to set a previous presentation mode of the visual target 25 that was appropriate for the subject eye as the predicted value Vp. Therefore, the ophthalmologic apparatus 10 can set the operation starting value Vs and the operation direction Do appropriate for the subject eye and can facilitate determination of an appropriate examination value through the examinee's rotation operation.

In the ophthalmologic apparatus 10, the control portion 14 is configured to set an effective adjustment range Re that is narrower than a maximum adjustment range Rm which is adjustable by using the rotation operation on the rotation operation portion 33. The effective adjustment range Re includes the predicted value Vp. Therefore, the ophthalmologic apparatus 10 can prevent a situation in which, due to the examinee's rotation operation, a value far from a value at which the visual target 25 is appropriately seen is determined as an appropriate examination value, thereby making it difficult to adjust to the appropriate value.

The ophthalmologic apparatus 10 further includes a determination operation portion 34 that is configured to determine the presentation mode of the visual target 25 indicated by using an operation position of the rotation operation portion 33. Therefore, the ophthalmologic apparatus 10 allows the examinee S to make an intuitive adjustment through the rotation operation, and to determine the examination value through the determination operation distinct from the rotation operation, thereby obtaining an examination value that appropriately reflects the examinee's intention.

The ophthalmologic apparatus 10 further includes the voice output portion 35 as an operation notification portion that is configured to notify an operation for an examination. The control portion 14 controls the voice output portion 35 to notify a first notification to prompt the rotation operation upon determining that the rotation operation has not been made on the rotation operation portion 33. The control portion 14 also controls the voice output portion 35 to notify a second notification to prompt a determination operation upon determining that the rotation operation has been made on the rotation operation portion 33, but the determination operation has not been made on the determination operation portion 34. Therefore, the ophthalmologic apparatus 10 can provide appropriate guidance to the examinee S in accordance with the rotation operation on the rotation operation portion 33 or the determination operation on the determination operation portion 34, thereby properly promoting the examination process.

In the ophthalmologic apparatus 10, the visual target presentation portion includes a visual target display device 12 that is configured to display the visual target 25, and an eye examination portion 21 that is configured to present the visual target 25 displayed by using the visual target display device 12 under any optical characteristic. The presentation mode of the visual target 25 is adjusted by varying an optical setting in the eye examination portion 21 relative to the subject eye. Therefore, the ophthalmologic apparatus 10 can adjust the presentation mode of the visual target 25 with a simple configuration and can support various examinations.

In the ophthalmologic apparatus 10, the visual target presentation portion 21, 12 includes a visual target display device 12 configured to display the visual target 25, and an eye examination portion 21 configured to present the visual target 25 displayed by using the visual target display device 12 under any optical characteristic. The visual target presentation portion 21, 12 adjusts the presentation mode of the visual target 25 by adjusting a posture of the visual target 25 displayed on the visual target display device 12. Therefore, the ophthalmologic apparatus 10 can adjust the presentation mode of the visual target 25 with a simple configuration and can support various examinations.

According to the ophthalmologic apparatus 10 as one embodiment of the ophthalmologic apparatus according to the present invention, appropriate examination results can be quickly obtained through the rotation operation on the rotation operation portion 33 done by the examinee S.

As described above, the ophthalmologic apparatus of the present invention has been explained based on the first embodiment. However, the specific configuration of the ophthalmologic apparatus is not limited to that of the first embodiment. Design changes or additions may be made as long as they do not depart from the gist of the invention defined in the claims.

For example, in the first embodiment, the visual target presentation portion is configured with the eye examination portion 21 and the visual target display device 12. However, the visual target presentation portion is not limited to the configuration of the first embodiment as long as it presents the visual target 25 to the subject eye and allows adjustment of the presentation mode under control of the control portion 14.

In the first embodiment, the separate controller 15 includes the rotation operation portion 33 and the determination operation portion 34. However, the rotation operation portion 33 and the determination operation portion 34 are not limited to those in the first embodiment. As long as the rotation operation portion 33 and the determination operation portion 34 allow the examinee S to make the rotation and determination operations, their locations and configurations may be set as desired. For example, the rotation operation portion 33 and the determination operation portion 34 may be implemented as a jog dial, which is a rotary selector operated simply by rotating and pressing it. The separate controller 15 may also have a simplified configuration including only the rotation operation portion 33 and the determination operation portion 34.

Furthermore, in the first embodiment, the rotation operation portion 33 is shown as one example of the increase and decrease operation portion configured to increase or decrease a numerical value through the rotation operation. However, the increase and decrease operation portion is not limited to that of the first embodiment. The increase and decrease operation portion may have any configuration as long as it allows an operation to increase or decrease a numerical value. Examples of other configurations include arrow icons and/or buttons indicating directions in which values increase or decrease, paired icons and/or buttons arranged left and right or up and down for selecting an increase or a decrease, and a wheel capable of forward and reverse rotation, like a mouse wheel.

The present invention further discloses the following.
(1) An ophthalmologic apparatus including:
   a visual target presentation portion that is configured to present a visual target to a subject eye and to adjust a presentation mode of the visual target;
   an increase and decrease operation portion that is configured to make an increase operation to increase a numerical value and a decrease operation to decrease the numerical value;
   a control portion that is configured to change the presentation mode of the visual target presented by the visual target presentation portion in response to the increase operation or the decrease operation on the increase and decrease operation portion; and
   a direction notification portion that is configured to notify an increase direction for the increase operation or a decrease direction for the decrease operation on the increase and decrease operation portion;
   wherein the control portion is further configured
   to set a predicted value for a presentation mode that is predicted to be appropriate for the subject eye;
   to set an operation starting value for a presentation mode that is different from the predicted value;
   to set the increase and decrease operation portion to the operation starting value; and
   to control the direction notification portion to notify an operation direction from the operation starting value toward the predicted value.
(2) The ophthalmologic apparatus according to above (1), wherein the increase and decrease operation portion comprises a rotation operation portion that is configured to increase or decrease the numerical value with a rotation operation on the rotation operation portion.
(3) The ophthalmologic apparatus according to above (1) or (2), wherein the control portion is configured to set, as the predicted value, a previous presentation mode that was appropriate for the subject eye.
(4) The ophthalmologic apparatus according to any one of the above (1) to (3), wherein the control portion is configured to set an effective adjustment range that is narrower than a maximum adjustment range, the maximum adjustment range being adjustable by using the increase and decrease operations on the increase and decrease operation portion, and wherein the effective adjustment range comprises the predicted value.
(5) The ophthalmologic apparatus according to any one of the above (1) to (4), further comprising a determination operation portion that is configured to determine the presentation mode indicated by an operation position of the increase and decrease operation portion.
(6) The ophthalmologic apparatus according to above (5), further comprising an operation notification portion that is configured to notify an operation for an examination, and
   wherein the control portion is configured
   to control the operation notification portion to notify a first notification to prompt the increase operation or the decrease operation upon a determination that the increase operation or the decrease operation has not been made on the increase and decrease operation portion, and
   to control the operation notification portion to notify a second notification to prompt a determination operation upon a determination that the increase operation or the decrease operation has been made on the increase and decrease operation portion, but the determination operation has not been made on the determination operation portion.
(7) The ophthalmologic apparatus according to any one of the above (1) to (6), wherein the visual target presentation portion comprises:
   a visual target display device that is configured to display the visual target; and
   an eye examination portion that is configured to present the visual target displayed on the visual target display device under any optical characteristic,
   wherein the visual target presentation portion is configured to adjust the presentation mode by varying an optical setting in the eye examination portion relative to the subject eye.
(8) The ophthalmologic apparatus according to any one of the above (1) to (6), wherein the visual target presentation portion comprises:
   a visual target display device that is configured to display the visual target; and
   an eye examination portion that is configured to present the visual target displayed on the visual target display device under any optical characteristic,
   wherein the visual target presentation portion is configured to adjust the presentation mode by varying a posture of the visual target displayed on the visual target display device.
(9) A method for controlling an ophthalmologic apparatus, the ophthalmologic apparatus comprising:
   a visual target presentation portion that is configured to present a visual target to a subject eye and to adjust a presentation mode of the visual target;
   an increase and decrease operation portion that is configured to make an increase operation to increase a numerical value and a decrease operation to decrease the numerical value;
   a control portion that is configured to change the presentation mode of the visual target presented by the visual target presentation portion in response to the increase operation or the decrease operation on the increase and decrease operation portion; and
   a direction notification portion that is configured to notify an increase direction for the increase operation or a decrease direction for the decrease operation on the increase and decrease operation portion;
   the method comprising:
      setting a predicted value for a presentation mode that is predicted to be appropriate for the subject eye;
      setting an operation starting value for a presentation mode that is different from the predicted value;
      setting the increase and decrease operation portion to the operation starting value; and
      controlling the direction notification portion to notify an operation direction from the operation starting value toward the predicted value.
(10) The method according to above (9), wherein the visual target presentation portion comprises:
   a visual target display device that is configured to display the visual target; and
   an eye examination portion that is configured to present the visual target displayed on the visual target display device under any optical characteristic,
   wherein the method further comprises adjusting the presentation mode by varying an optical setting in the eye examination portion relative to the subject eye.
(11) The method according to above (9), wherein the visual target presentation portion comprises:
   a visual target display device that is configured to display the visual target; and
   an eye examination portion that is configured to present the visual target displayed on the visual target display device under any optical characteristic,
   wherein the method further comprises adjusting the presentation mode by varying a posture of the visual target displayed on the visual target display device.

### List of Reference Signs

- 10: ophthalmologic apparatus
- 12: visual target display device (an example of visual target presentation portion)
- 14: control portion
- 21: eye examination portion (an example of visual target presentation portion)
- 25: visual target
- 33: rotation operation portion (an example of increase and decrease operation portion)
- 34: determination operation portion
- 35: voice output portion (an example of direction notification portion)
- Do: operation direction
- Re: effective adjustment range
- Rm: maximum adjustment range
- Vp: predicted value
- Vs: operation starting value

## Claims

1. An ophthalmologic apparatus (10) comprising:
a visual target presentation portion (21, 12) that is configured to present a visual target (25) to a subject eye and to adjust a presentation mode of the visual target (25);
an increase and decrease operation portion (33) that is configured to make an increase operation to increase a numerical value and a decrease operation to decrease the numerical value;
a control portion (14) that is configured to change the presentation mode of the visual target (25) presented by the visual target presentation portion (21, 12) in response to the increase operation or the decrease operation on the increase and decrease operation portion (33); and
a direction notification portion (35) that is configured to notify an increase direction for the increase operation or a decrease direction for the decrease operation on the increase and decrease operation portion (33);
wherein the control portion (14) is further configured
to set a predicted value (Vp) for a presentation mode that is predicted to be appropriate for the subject eye;
to set an operation starting value (Vs) for a presentation mode that is different from the predicted value (Vp);
to set the increase and decrease operation portion (33) to the operation starting value (Vs); and
to control the direction notification portion (35) to notify an operation direction (Do) from the operation starting value (Vs) toward the predicted value (Vp).

2. The ophthalmologic apparatus (10) according to claim 1, wherein the increase and decrease operation portion (33) comprises a rotation operation portion (33) that is configured to increase or decrease the numerical value with a rotation operation on the rotation operation portion (33).

3. The ophthalmologic apparatus (10) according to claim 1 or 2, wherein the control portion (14) is configured to set, as the predicted value (Vp), a previous presentation mode that was appropriate for the subject eye.

4. The ophthalmologic apparatus (10) according to any one of claims 1 to 3, wherein the control portion (14) is configured to set an effective adjustment range (Re) that is narrower than a maximum adjustment range (Rm), the maximum adjustment range (Rm) being adjustable by using the increase and decrease operations on the increase and decrease operation portion (33), and
wherein the effective adjustment range (Re) comprises the predicted value (Vp).

5. The ophthalmologic apparatus (10) according to any one of claims 1 to 4, further comprising a determination operation portion (34) that is configured to determine the presentation mode indicated by an operation position of the increase and decrease operation portion (33).

6. The ophthalmologic apparatus (10) according to claim 5, further comprising an operation notification portion (35) that is configured to notify an operation for an examination, and
wherein the control portion (14) is configured
to control the operation notification portion (35) to notify a first notification to prompt the increase operation or the decrease operation upon a determination that the increase operation or the decrease operation has not been made on the increase and decrease operation portion (33), and
to control the operation notification portion (35) to notify a second notification to prompt a determination operation upon a determination that the increase operation or the decrease operation has been made on the increase and decrease operation portion (33), but the determination operation has not been made on the determination operation portion (34).

7. The ophthalmologic apparatus according to any one of claims 1 to 6, wherein the visual target presentation portion (21, 12) comprises:
a visual target display device (12) that is configured to display the visual target (25); and
an eye examination portion (21) that is configured to present the visual target (25) displayed on the visual target display device (12) under any optical characteristic,
wherein the visual target presentation portion (21, 12) is configured to adjust the presentation mode by varying an optical setting in the eye examination portion (21) relative to the subject eye.

8. The ophthalmologic apparatus according to any one of claims 1 to 6, wherein the visual target presentation portion (21, 12) comprises:
a visual target display device (12) that is configured to display the visual target (25); and
an eye examination portion (21) that is configured to present the visual target (25) displayed on the visual target display device (12) under any optical characteristic,
wherein the visual target presentation portion (21, 12) is configured to adjust the presentation mode by varying a posture of the visual target (25) displayed on the visual target display device (12).

9. A method for controlling an ophthalmologic apparatus, the ophthalmologic apparatus comprising:
a visual target presentation portion (21, 12) that is configured to present a visual target (25) to a subject eye and to adjust a presentation mode of the visual target (25);
an increase and decrease operation portion (33) that is configured to make an increase operation to increase a numerical value and a decrease operation to decrease the numerical value;
a control portion (14) that is configured to change the presentation mode of the visual target (25) presented by the visual target presentation portion (21, 12) in response to the increase operation or the decrease operation on the increase and decrease operation portion (33); and
a direction notification portion (35) that is configured to notify an increase direction for the increase operation or a decrease direction for the decrease operation on the increase and decrease operation portion (33);
the method comprising:
setting a predicted value (Vp) for a presentation mode that is predicted to be appropriate for the subject eye;
setting an operation starting value (Vs) for a presentation mode that is different from the predicted value (Vp);
setting the increase and decrease operation portion (33) to the operation starting value (Vs); and
controlling the direction notification portion (35) to notify an operation direction (Do) from the operation starting value (Vs) toward the predicted value (Vp).

10. The method according to claim 9, wherein the visual target presentation portion (21, 12) comprises:
a visual target display device (12) that is configured to display the visual target (25); and
an eye examination portion (21) that is configured to present the visual target (25) displayed on the visual target display device (12) under any optical characteristic,
wherein the method further comprises adjusting the presentation mode by varying an optical setting in the eye examination portion (21) relative to the subject eye.

11. The method according to claim 9, wherein the visual target presentation portion (21, 12) comprises:
a visual target display device (12) that is configured to display the visual target (25); and
an eye examination portion (21) that is configured to present the visual target (25) displayed on the visual target display device (12) under any optical characteristic,
wherein the method further comprises adjusting the presentation mode by varying a posture of the visual target (25) displayed on the visual target display device (12).
